# EUROPEAN PATENT SPECIFICATION

(11) **EP 3 351 665 B1**
(45) Date of publication and mention of the grant of the patent: **03.06.2020**
(21) Application number: 18158974.8
(22) Date of filing: 01.11.2012
(51) Int. Cl.: A61F 13/00, A61L 15/42, A61L 15/60, B32B 5/24, D06N 3/00

(54) **GELLING WOUND DRESSINGS**
GELIERENDE WUNDVERBÄNDE
PANSEMENTS GELIFIANTS

(30) Priority: 01.11.2011 GB 201118857; 29.06.2012 GB 201211540
(43) Date of publication of application: 25.07.2018
(62) Divisional of application: 12791837.3
(73) Proprietor: Brightwake Limited, Nottingham, Nottinghamshire NG17 7JZ (GB)
(72) Inventor: COTTON,, Stephen, Nottingham, Nottinghamshire NG15 8EQ (GB)
(74) Representative: Adamson Jones

(56) References cited:
- WO-A1-95/19795
- WO-A1-2005/023323
- WO-A1-2005/079718
- WO-A1-2011/058311
- US-A1- 2007 255 194
- US-A1- 2011 213 286
- DOWSETT CAROLINE: "Managing wound exudate: role of Versiva XC gelling foam dressing", BRITISH JOURNAL OF NURSING, ALLEN, LONDON, GB, vol. 17, no. 11, 1 June 2008 (2008-06-01), pages S38, 40-S42, XP008161391, ISSN: 0966-0461, DOI: 10.12968/BJON.2008.17.SUP5.29650

## Description

The present invention relates to wound dressings. More particularly, the present invention relates to a gelling foam dressing, comprising a sheet of gelling foam laminated with a scrim comprising gelling fibres and non-gelling fibres.

Different types of wound dressing are required to meet different clinical needs. However, a common requirement for wound dressings is that they are able to absorb exudate from a wound, while retaining sufficient structure that they can be easily removed from the wound after use without tearing or disintegrating. If a wound dressing cannot be cleanly removed from a wound then a patient will suffer additional trauma. In addition, if the wound dressing breaks apart or disintegrates during removal then fragments of the dressing may be left in the wound, inhibiting healing. For some wounds in particular, for example sinus wounds, it is essential that all of the material is removed when the wound dressing is changed, as cases of giant cell foreign body reaction have been reported where fragments of alginate dressings have remained in the wound.

In addition to the need to retain structural integrity, it is important for many applications that a wound dressing is able to absorb a significant amount of liquid. During the healing process, wounds produce exudate. This is absorbed by the dressing in order to keep the wound clean and promote healing. A determining factor in how regularly a dressing needs to be changed is how quickly the dressing becomes saturated with exudate. Infrequent dressing changes are preferable as changing a dressing can aggravate a wound, as well as causing pain and/or discomfort for the patient.

Gelling fibres such as alginate or pectin fibres are known for use in wound dressings. They have a greater capacity for absorbing liquid than standard textile fibres and, on absorbing liquid, they become moist and slippery. This prevents the dressing from adhering to the wound and therefore makes removal of the dressing easier. However, it also causes the gelling fibres to lose structural integrity, making them more difficult to handle, and more difficult to remove cleanly from a wound. In addition, the gelling fibres themselves may be brittle, making them difficult to work with in the production of wound dressings. Hence, despite their advantages, it has generally only been possible to produce wound dressings entirely out of gelling fibres when the wound dressing is formed of a non-woven fabric. However, non-woven fabrics generally have a low tensile strength, resulting in a loss of integrity when the dressing is saturated with liquid. In addition, non-woven fabrics may shed fibres, a trait which is extremely undesirable in a wound dressing. Efforts have been made to overcome these limitations by producing wound dressings formed of a combination of gelling fibres and non-gelling fibres.

EP0925396 is concerned with the provision of a wound dressing which is absorbent but non-adherent. The wound dressing described comprises a knitted support yarn (which is substantially free of gelling fibres) and an in-laid yarn containing gelling fibres. The support yarn is typically a cellulosic fibre such as viscose rayon or cotton, and the gelling fibres are preferably sodium carboxymethylcellulose fibres.

EP0927013 describes the use of a mixture of modified cellulose gelling fibres and another form of gelling fibre (such as alginate) to form a wound dressing. The two types of gelling fibre may be spun or twisted together to form a yarn and then knitted or woven to form a bandage or stocking. The wound dressing may also comprise other fibres such as textile fibres, for example, viscose rayon.

EP0740554 describes an absorbent, non-adherent wound dressing comprising a mixture of textile fibres and gel forming fibres which may be spun or twisted to form a yarn and then knitted or woven into a bandage or stocking. The wound dressing described preferably comprises 80% by weight of textile fibres and 20% by weight of gelling fibres.

WO 2005/079718 describes a multi-layered wound dressing comprising a transmission layer and an absorbent core. The absorbent core may comprise a combination of gel-forming and textile fibres, and the transmission layer may be a film/foam laminate, e.g., a polyurethane film/foam laminate. The transmission layer must have a high moisture-vapour transmission rate.

WO 2011/058311 discloses a component of a wound dressing which comprises a wound contacting layer of gel-forming fibres bound to a foam layer. The wound contacting layer may additionally comprise textile fibres. Preferably, the foam is a hydrophilic foam, with polyurethane foam given as an example, the purpose of the foam layer being that it absorbs exudate but readily releases it through moisture vapour transmission.

The proportion of gelling fibres used in wound dressings of the prior art has been limited due the propensity of gelling fibres to lose their integrity when saturated with liquid, and concerns that this would lead to an unacceptable loss of integrity of the wound dressing as a whole. Typically, less than a third by weight of wound dressings such as those described above comprises gelling fibres. This limits the capacity of the wound dressing to absorb exudate, and means that the dressing will require changing frequently.

In addition to the anticipated problems of loss of integrity, the brittle nature of gelling fibres raised concerns that yarns which contain a high proportion of gelling fibres would break and fragment during production of wound dressings, particularly in processes which involve placing stress on the fibres such as knitting and weaving. If that happened, the wound dressing produced would be weaker and lack integrity, making it more likely to fragment in the wound.

However, it has now surprisingly been found that when gelling fibres are blended with non-gelling fibres, the gelling fibre can be used in high proportions, with only a low proportion of non-gelling fibre being required to maintain the structural integrity of the dressing in use.

Disclosed herein is among others a knitted structure for use as, or as a component of, a wound dressing, the structure being knitted from yarn comprising a blend of gelling fibres and non-gelling fibres, wherein the yarn comprises at least 50% w/w gelling fibres.

Such a knitted structure overcomes or substantially mitigates some or all of the disadvantages of the prior art. Advantageously, it comprises a large proportion of gelling fibres, thereby providing a high capacity for absorbing wound exudate. This means that the wound dressing can be changed less frequently, reducing discomfort and pain for the patient. It has been surprisingly found that the combination of gelling fibre and non-gelling fibre in a blended yarn produces a strong, flexible yarn that can be knitted, despite the relatively low proportion of non-gelling fibre. Knitted materials have a high tensile strength whilst retaining some stretch and movement. These characteristics are advantageous in a wound dressing where the movement of the patient to whom the dressing has been applied may cause the dressing to distort. The flexible nature of the knitted structure allows for distortion without disruption or tearing of the dressing.

Furthermore, it has been found that a knitted structure comprising a blended yarn as disclosed herein retains its structural integrity after use in a wound dressing. Even when saturated with liquid, the structure retains sufficient integrity that it can be easily removed from the wound with little or no breakage or disintegration. The retained structure of the wound dressing is such that even rope dressings of the type commonly used to pack sinus wounds may be removed easily and without breakage.

Also disclosed herein is a yarn comprising a blend of gelling fibres and non-gelling fibres, wherein the yarn comprises at least 50% w/w gelling fibres.

Advantageously, the blended yarn disclosed herein contains a high proportion of gelling fibres, making it useful in the manufacture of wound dressings. Further, it has been surprisingly found that the combination of a high proportion of gelling fibres with a small proportion of non-gelling fibres in a blended yarn produces a strong and flexible yarn that can be knitted without substantial breakage or weakening, and which retains its integrity when saturated with liquid.

The knitted structure disclosed herein may itself be used as a simple form of wound dressing, eg in applications for which non-woven dressings of alginate or CMC are conventionally used. For such applications, the structure takes the form of a simple sheet or ribbon of knitted fabric. Sheets may be square, rectangular, circular, ovoid, or may have any other suitable shape.

In other applications, however, the knitted structure is a component of a composite wound dressing. In one such application, the knitted structure is incorporated into a foam dressing, in particular a gelling foam dressing. Such dressings formed from alginate materials are already known. In the production of a conventional such dressing, an aqueous solution of alginate is cast into a mould and allowed to gel. Water is then removed from the resultant hydrogel, eg by heating or more commonly by lyophilisation, to form a sheet of foam. The foam may then be used in a manner similar to a non-woven alginate dressing, ie the foam sheet is placed on a wound and absorbs wound exudate, the foam thereby forming a gel.

A disadvantage of known forms of gelling foam dressings is that they tend to completely lose their integrity in use, making complete removal of the dressing difficult or impossible.

According to the present invention, a gelling foam dressing is reinforced with a scrim comprising gelling fibres and non-gelling fibres. Thus, the invention provides a gelling foam dressing comprising a sheet of gelling foam laminated with a scrim comprising gelling fibres and non-gelling fibres. The scrim may be a knitted structure according to the first aspect of the invention, ie a knitted structure knitted from yarn comprising a blend of gelling fibres and non-gelling fibres, wherein the yarn comprises at least 50% w/w gelling fibres. In other embodiments, the scrim may be woven.

The gelling foam dressing according to the invention is advantageous primarily in that the scrim reinforces the foam, and preserves the integrity of the dressing sufficiently for the dressing to be easily and substantially completely removed from a wound site, even after the dressing has absorbed substantial quantities of wound exudate and has become gel-like.

Also disclosed herein are three-dimensional textile materials comprising gelling fibres, which textile materials are useful as, or as components of, wound dressings. One form of wound therapy in which such dressings may be of particular utility is negative pressure wound therapy (NPWT), a technique designed to promote the formation of healthy granulation tissue and so promote wound healing that is also referred to as vacuum-assisted closure. "Negative pressure" in this context means pressure that is lower than ambient air pressure. NPWT may be used to enhance healing in chronic, acute and hard-to-heal wounds. In large, open wounds, a packer is required to fill the wound cavity and to support and aid the positioning of a drainage tube. An occlusive dressing is used to cover the wound bed and form a seal or gasket beneath which the vacuum is formed. The dressing must be adhered to viable dermal tissue surrounding the wound and pinched around the drainage tube to ensure a proper seal. Negative pressure is then applied and the wound fluid is collected. If a suitable packer is not utilised, the dressing is sucked into the wound cavity and thus does not form an effective seal.

The wound packer must be non-adhesive and porous, and must contact the entire wound. Conventionally, open cell foams or gauzes are used to pack the wound cavity.

When gauze is used, it is typically applied as a single layer. A drain is placed on the gauze and then a second piece of gauze is placed over the drain, creating a "gauze-sandwich". The gauze may be impregnated, eg with antimicrobials.

More commonly, foams such as polyurethane foam and polyvinylalcohol (PVA) foam are used as packing materials in negative pressure wound therapy. PVA foam has smaller pores compared to polyurethane foam and, because of its higher density, requires higher negative pressures than PVA to work effectively. Foams are usually cut to fit the size and shape of the wound, and more than one piece of foam can be used if necessary. However, each piece of foam must come into contact with another piece of foam to achieve uniform compression when negative pressure is applied.

However, all of the packing materials currently available have associated difficulties and risks. In particular, the packing material may stick to the wound, causing pain to the patient during dressing. There is also a risk that a piece of foam or gauze fibre will become detached and remain in the wound, leading to a foreign body reaction or the formation of an abscess. The packing materials may also become clogged, obstructing the passage of wound exudate and/or inhibiting the application of negative pressure to the wound.

It has now been found that three-dimensional textile materials and wound dressings incorporating the same, in which the textile material comprises gelling fibres overcome or substantially mitigate some or all of the above-mentioned and/or other disadvantages of the prior art.

By "three-dimensional textile material" is meant in the context of the invention a textile material with a structure such that the textile material has a substantial thickness, ie the textile material is bulky in the dimension that is transverse to the plane of the textile material. Generally, such a textile material will be compressible, such that its thickness may be reduced substantially by the application of mechanical force, eg by a factor of 2 or more, eg a factor of 5, or 10, or 20, or more.

By "gelling fibre" is meant in relation to all aspects of the invention fibres that are capable of absorbing aqueous fluid, such as wound exudate, and which on absorbing liquid become gel-like, moist and slippery. The gelling fibres may have an absorbency of at least 2 grams 0.9% saline solution per gram of fibre, as measured by the free swell absorbency test (ie dispersing a known dry weight of fibre in the test liquid (saline) for sufficient time for the fibre to absorb liquid, removing the excess liquid by vacuum filtration, and measuring the increase in weight of the fibre). The absorbency may be considerably higher, eg at least 5g/g, or at least 10g/g, or at least 15g/g, or at least 25g/g.

Methods for producing yarn from blended fibres are known in the art, and the yarn disclosed herein may be made by blending gelling fibres and non-gelling fibres by any suitable method. Commonly, short lengths of fibre (staple fibres) are blended together before being spun into a yarn. Preferably, the staple fibres used in production of the yarn have a length greater than 30mm. More preferably, they have a length greater than 40mm. The length of the staple fibres may be less than 100mm, eg 30-100mm or40-100mm. The lengths of the staple fibres may be variable, in which case the mean length may be in the ranges specified above.

The gelling fibres may be any suitable gelling fibre known in the art, including pectin fibres, alginate fibres, fibres made from alginate and another polysaccharide, chitosan fibres, hyaluronic acid fibres, fibres of other polysaccharides or derived from gums, or chemically-modified cellulosic fibres, eg carboxymethyl cellulose (CMC). The gelling fibres may be a combination or blend of different gelling fibres.

Currently preferred gelling fibres for all aspects of the invention are alginate fibres and pectin fibres.

Alginates are high molecular weight, hydrophilic polymers, which are derived from seaweed and which form a gel on contact with aqueous fluids. Their hydrophilic nature encourages the absorption of liquid such as wound exudate, making them extremely useful in wound dressings.

The alginate polymer is formed of two basic monomeric units, mannuronic acid and guluronic acid. Differing proportions of these units in the polymer alter the properties of the alginate. In addition to this, alginate polymers are associated with cations, and are normally produced in the form of sodium alginate, calcium alginate or a sodium/calcium alginate mix. Other forms, such as potassium alginate, are also known.

The nature of the cation which is associated with the alginate polymer changes the properties of the alginate. For example, sodium alginate is water soluble, whereas calcium alginate is not. By altering the alginate used in a wound dressing it is therefore possible to ensure that the final dressing displays the desired characteristics.

Alginate fibres are produced in a number of forms, but most commonly in the form of sodium alginate, calcium alginate or a sodium/calcium alginate mix. Other forms, such as potassium alginate, are also known. The different alginates have different characteristics, and it is therefore possible to vary the relative proportions of the different alginates used in order to obtain the desired properties in the yarn. For example, sodium alginate is water-soluble, and a yarn produced using a high proportion of sodium alginate may therefore lose some of its structural integrity when saturated with water. In contrast to this, calcium alginate is not water-soluble. On contact with wound exudate, sodium ions in the wound exudate exchange with calcium ions in the alginate, and it is the presence of sodium ions in the alginate that then causes the formation of a gel. Alginate fibres for use in a blended yarn according to the present invention may be calcium alginate, sodium alginate, or mixed calcium/sodium alginate. Preferably, alginate fibres for use in the present invention are calcium alginate.

Pectins are a family of complex polysaccharides comprising 1,4-linked α-D-galactosyluronic residues, found primarily in the cell walls of terrestrial plants. Pectins can be separated into two main groups which have different gelling properties: low-methoxy and high-methoxy pectins. Low-methoxy pectins are pectins in which less than half the carbonyl groups in the chain of galacturonic residues are esterified with methanol. Low-methoxy pectins can form a gel in the presence of divalent cations (eg calcium), due to non-covalent ionic interactions between blocks of galacturonic acid residues and the divalent ion. High-methoxy pectins are those in which more than half of the carbonyl groups have been esterified with methanol. Such pectins can gel in the presence of sugar and acid, forming two-dimensional networks of pectin molecules in which the solvent (water) is immobilised with the sugar and acid co-solutes.

Another class of gelling fibres that are known to be useful in absorbent wound dressings are those made from chemically-modified cellulose. In particular, carboxymethylated cellulose fibres may be used, eg in the form of sodium carboxymethyl cellulose. Such fibres preferably have a degree of substitution of at least 0.2 carboxymethyl groups per glucose unit, or at least 0.3 or at least 0.5. Methods for producing gelling fibres are known in the art, and any suitable method may be employed to produce fibres for use in the present invention. For example, calcium alginate fibres may be produced by solvent-spinning a sodium alginate solution through a solution of calcium ions. Similarly, pectin fibres may be produced by solvent-spinning a solution of the pectin polymer into a bath of a water-miscible organic solvent. The fibres may be further processed by any suitable method known in the art, including washing, crimping, carding, spinning and/or cutting.

Knitted structures as disclosed herein may take various forms, eg sheets, bandages, tubular bandages, ropes, or any other shape which is suitable for use as a wound dressing. Many such structures will be essentially two-dimensional, having a thickness that is considerably less than the other dimensions. Such structures are typically sheet-like, having a square or rectangular shape, or being shaped for conformity with a particular area of the body. Other structures may be formed in three-dimensional shapes, eg tubular dressings.

The knitted structure may also be produced in a form suitable for use as wound packing material, ie a material used not to cover a wound, but to fill a cavity or sinus would. For such applications, the knitted structure may be produced as relatively small, discrete wound packing elements or as a band or ribbon.

The non-gelling fibres may be any suitable fibres known in the art, or may be a mixture of two or more non-gelling fibres. The non-gelling fibres may be textile fibres, and may be natural, eg cotton, may be natural fibres which have been modified eg cellulosic fibres such as viscose or lyocell (sold under the trade name TENCEL®), or they may be synthetic, eg polyester, polypropylene or polyamide. Different fibres have different characteristics in terms of tensile strength and absorbency, and appropriate non-gelling fibres may be chosen according to the desired characteristics of the wound dressing. In addition, a combination of two or more non-gelling fibres may be used in order to achieve the desired characteristics. Preferably, the non-gelling fibres are natural fibres which have been modified. More preferably, the non-gelling fibres are cellulosic fibres.

Thus a yarn may comprise a combination of alginate fibres and cellulosic fibres. In such cases, the yarn preferably comprises a combination of calcium alginate fibres and cellulosic fibres. More preferably, the yarn comprises calcium alginate and viscose, or calcium alginate and lyocell.

In other cases, the yarn may comprise a combination of pectin fibres and cellulosic fibres. In such cases, the yarn preferably comprises a combination of pectin fibres and viscose, or pectin fibres and lyocell.

The ratio of gelling fibre to non-gelling fibre in the yarn can vary between quite wide limits, provided that the proportion of gelling fibre (by weight) in the blend is at least 50%. Preferably, the gelling fibre comprises 50-98% w/w of the blend. The gelling fibre may comprise 60-98% w/w of the blend, or 70-98% w/w of the blend. Preferably, the non-gelling fibre comprises 2-49% w/w of the blend. The non-gelling fibre may comprise 5-49% w/w of the blend, or 10-30% of the blend. Thus, the ratio of gelling fibre to non-gelling fibre may be from 98:2 to 51:49. The ratio of gelling fibre to non-gelling fibre may be from 98:2 to 60:40, more preferably from 98:2 to 70:30. For example, the ratio of gelling fibre to non-gelling fibre may be approximately 60:40, 65:35, 70:30, 75:25, 80:20, 85:15, 90:10, 95:5 or 98:2. The proportions and ratios set out in the preceding paragraph may in general apply to any combinations of gelling and non-gelling fibres, eg combinations in which the gelling fibres are alginate fibres, pectin fibres or modified cellulose fibres, and combinations in which the non-gelling fibres are natural, eg cotton, natural fibres which have been modified eg cellulosic fibres such as viscose or lyocell, or synthetic fibres, eg polyester, polypropylene or polyamide. The same is true of the staple fibre lengths and absorbencies described above.

The structure according to one aspect of the present disclosure is knitted.

Knitting is a process whereby fabric is formed by the interlocking of loops of yarn. A variety of knitting techniques are known in the art, and are suitable for use in this aspect of the present disclosure.

Preferably, the structure is knitted using a double needle-bed. A double needle-bed produces a knitted fabric which has higher strength, and greater bulk, than knitted fabrics produced by other methods. This ensures that the structure has high structural integrity, and aids in the retention of integrity even after use.

Knitted fabrics may warp-knitted or weft-knitted. In warp-knitted fabrics, rows of loops are made along the length of the fabric (the warp). A common way to achieve this is to feed numerous lengths of yarn simultaneously to rows of individual needles. In weft-knitted fabrics, the loops are made horizontally across the fabric (the weft), normally using a single yarn, and the stitches are formed by the interlocking of the loops with loops of the rows above and below. Preferably, knitted structures according to the present disclosure are warp-knitted.

Methods for producing yarn from blended fibres are known in the art, and the yarn according to the present disclosure may be made by blending the gelling fibres and non-gelling fibres by any suitable method.

The yarn may be of any suitable type known in the art. In particular, it may be desirable for a product produced using the yarn to have structure, in order that it maintains its shape and form. Such a product may be produced through the use of a textured yarn, eg an air-intermingled yarn, false twist yarn, multiple-ply yarn, KDK (knit-deknit) yarn or other similar yarn. The yarn according to the present invention may therefore be a textured yarn.

The gelling foam dressing of the invention may comprise a single sheet of foam, to one face of which the scrim is laminated. Such embodiments may be manufactured by
a) casting a solution of the gelling material, eg alginate, into a mould,
b) allowing the solution to partially cure,
c) laying the scrim on top of the partially-cured solution,
d) allowing the solution to cure further to form a hydrogel, and
e) removing water from the hydrogel to form a foam sheet bonded to the scrim.

In other embodiments, the scrim is sandwiched between layers of foam, for example by
a) casting a first quantity of solution of the gelling material, eg alginate, into a mould,
b) allowing the solution to partially cure,
c) laying the scrim on top of the partially-cured solution,
d) casting a further quantity of solution of the gelling material onto the scrim,
e) allowing the solution to cure further to form a hydrogel, and
f) removing water from the hydrogel to form foam sheets bonded to both sides of the scrim.

The gelling material used in the gelling foam dressing may be any of those described above. However, the currently most preferred gelling material is alginate.

The solution of alginate that is used will typically comprise sodium alginate and calcium ions (or other metal ions, eg zinc, aluminium, silver or copper) to react with the alginate to bring about formation of a hydrogel. In order to allow the solution to be cast into a mould without premature precipitation of insoluble calcium alginate, it may be desirable or necessary to include in the solution a sequestering agent such as ethylenediamine tetra-acetic acid (EDTA) or sodium citrate (citric acid, trisodium salt dihydrate) to retard the precipitation of the insoluble metal ion alginate gel for a period of time that permits the sodium alginate and Ca²⁺ ion-containing solution to be poured into the mould, so that gelation take place after a suitable period of time and the insoluble alginate gel retains the form of the mould into which it is poured. Another method which may be used is to add an insoluble calcium salt, such as calcium carbonate, to a solution of sodium alginate, which is then poured into the mould. The calcium ion reacts very slowly with the alginate moiety and consequently precipitation of the calcium alginate gel is delayed, allowing gelation to occur and the hydrogel so formed to take the form of the mould.

Removal of water from the foam sheet may be carried out by conventional means, eg by lyophilisation or by heating.

The gelling materials used in the gelling foam and in the scrim that reinforces the foam are most conveniently, though not necessarily, of the same kind. For instance, the foam may be an alginate foam and the scrim may be formed from yarns comprising alginate fibres and a non-gelling fibre. The non-gelling fibre component of the scrim provides the integrity necessary to enable removal of the dressing from a wound, even after gelling has occurred, and the gelling fibre component facilitates bonding of the scrim to the foam.

The three-dimensional textile material disclosed herein is preferably a "double-faced" or "spacer" fabric. Spacer fabrics comprise two distinct face layers of fabric joined together by a connecting layer. The connecting layer most commonly has the form of connecting pile threads that extend between the two outer face layers. The two outer face layers may be the same or different. Most preferably, each face layer will have a knitted structure with a regular array of openings. The size and/or arrangement of the openings in the two face layers may be the same or different.

The connecting layer comprises threads, eg monofilaments or yarns, which connect the two face layers. Typically the spacer fabric is knitted such that the connecting threads are arranged substantially perpendicular to the two face layers. The distance between the two face layers, ie the thickness of the spacer fabric, is determined by the length of the connecting threads. The properties of the spacer fabric may also be varied by appropriate choice of the type of connecting thread and by varying the density of the connecting threads.

The structure of this material makes it particularly advantageous for use in the field of wound care. The open structure of the three-dimensional textile material allows a ready flow of air through the material, and means that it is less likely than conventional dressings and packing materials to become clogged with wound exudate; this is particularly important in negative pressure wound therapy. The use of gelling fibres in the material results in a high capacity for absorbing fluid, particularly wound exudate. This, coupled with the open structure of the material, enables good transfer of exudate away from a wound. The highly absorbent, non-adherent nature of the fabric also means that a wound dressing comprising this material will require changing less frequently, and will be easy to remove from the wound, reducing trauma and pain for the patient. The gelling fibres additionally help to maintain a moist wound environment which aids healing.

The three-dimensional textile material may be formed in a knitting operation, using a yarn as described above, ie a yarn comprising a blend of gelling fibres and non-gelling fibres, wherein the yarn comprises at least 50% w/w gelling fibres. The proportion of non-gelling fibres may be less than 40% w/w of the textile material, preferably less than about 20% w/w of the textile material, more preferably the proportion of non-gelling fibres is less than about 15% w/w, or less than about 10% w/w or less than about 5% w/w of the textile material. The proportion of gelling fibres may thus be between 50% and 100% w/w of the textile material, or between 80% and 100% w/w of the textile material, or between 90% and 100% w/w of the textile material, or between 50% and 95% w/w, between 80% and 95% w/w, or between 85% and 95% w/w. Alternatively, in this aspect of the invention the three-dimensional textile material may consist entirely of gelling fibres, ie gelling fibres comprise 100% w/w of the three-dimensional textile material.

Three-dimensional textile materials disclosed herein preferably have a thickness of at least 1mm, more preferably at least 1.5mm, and more preferably at least 2mm. Three-dimensional textile materials suitable for use in the present invention preferably have a thickness of up to 60mm, more preferably a thickness of up to 20mm, more preferably a thickness of up to 15mm, and most preferably a thickness of up to 10mm. Accordingly, three-dimensional textile materials preferably have a thickness in the range of 1mm to 60mm, more preferably 1.5mm to 20mm, more preferably 2mm to 15mm and most preferably 2mm to 10mm.

The gelling fibres and non-gelling fibres may be blended together prior to production of the three-dimensional textile material. The blended fibres may be used in the production of the entire material such that the gelling fibres and non-gelling fibres are evenly distributed throughout the material. Alternatively, the non-gelling fibres may only be incorporated into part of the fabric. For example, the non-gelling fibres may comprise all or part of the first outer face layer, the second outer face layer and/or the connecting threads of a spacer fabric.

As in other aspects of the disclosure the non-gelling fibres may be any suitable fibres known in the art, or may be a mixture or blend of two or more non-gelling fibres. The non-gelling fibres may be textile fibres, and may be natural fibres, eg cotton, natural fibres which have been modified, eg cellulosic fibres such as viscose or lyocell, or they may be synthetic, eg polyester, polypropylene or polyamide. Different fibres have different characteristics in terms of tensile strength and absorbency, and appropriate non-gelling fibres may be chosen according to the desired characteristics of the material. In addition, a combination of two or more non-gelling fibres may be used in order to achieve the desired characteristics. Preferably, the non-gelling fibres are natural fibres which have been modified. More preferably, the non-gelling fibres are cellulosic fibres. Most preferably, the non-gelling fibres are lyocell, which are sold under the trade name TENCEL®.

When the gelling fibres absorb liquid and gel, they may lose their structural integrity. The addition of a proportion of non-gelling fibres to the three-dimensional textile material promotes retention of the structural integrity of the fabric, whilst still retaining the high absorbency and non-adherent properties of the gelling fibres. This enables the dressing to be easily removed from a wound, without the risk of leaving fragments of fibre in the wound.

The above-described three-dimensional textile material has particular application in wound dressings.

Hence, according to a further aspect of the disclosure there is provided a wound dressing comprising a three-dimensional textile material, said three-dimensional textile material comprising gelling fibres.

Wound dressings comprising a three-dimensional textile material according to the disclosure may have various forms. As a rule, the three-dimensional textile material will constitute the wound-contacting component of the dressing.

In some cases, the wound dressing may consist entirely or substantially of the three-dimensional textile material. In other words, the dressing may be made up largely or entirely of a piece of the textile material, eg a square, rectangular or otherwise suitably shaped piece of the material. In such a case, the dressing may be used in a similar manner to a conventional gelling dressing such as a dressing of alginate or carboxymethylcellulose fibres, which typically have the form of non-woven pads. Compared with such conventional dressings, a dressing according to the invention may be characterised by a greater capacity to transmit wound exudate and so such dressings may advantageously be used in conjunction with absorbent bodies (eg foam or gauze pads) placed on the opposite side of the dressing to the wound. The three-dimensional textile material may also be used as a wound packing material, and so may be produced in the form of relatively small, discrete wound packing elements or as a band or ribbon.

In other cases, the wound dressing is a composite dressing comprising the three-dimensional textile material together with other components.

Composite wound dressings according to this aspect of the disclosure typically comprise a backing layer, which forms a barrier between the wound and the surrounding atmosphere. Any suitable material known in the art may be used for the backing layer.

The backing layer will generally be impermeable to wound exudate and other liquids, but is preferably permeable to air and moisture vapour. In particular, the backing layer preferably exhibits a relatively high moisture vapour transmission rate (MVTR). The MVTR of the backing layer may be at least 300g/m²/24h, more suitably at least 500g/m²/24h and preferably at least 700g/m²/24h at 37°C and 100% to 10% relative humidity difference.

The backing layer is most preferably a plastics film having the desired characteristics. The backing layer may be a polyurethane film.

The backing layer may be larger in size than the three-dimensional textile material, such that it extends beyond the edge of the three-dimensional textile material on one or more sides. Preferably, the backing layer extends beyond the edge of the three-dimensional textile material on all sides, forming a border around the three-dimensional textile material.

The backing layer may carry an adhesive on its underside. Where the backing layer forms a border around the three-dimensional textile material, the adhesive may serve to adhere the wound dressing to the patient's skin around the wound. The size of the dressing will generally be chosen such that the three-dimensional textile material overlies the wound and the border contacts healthy skin around the wound. Suitable skin contact adhesives for wound dressings are known, and any suitable adhesive known in the art may be used in the present invention. For example, the adhesive may be an acrylic adhesive, hydrocolloid adhesive, polyurethane adhesive, hydrogel or soft silicone adhesive.

Soft silicone adhesives offer numerous advantages. Most preferably, the soft silicone adhesive is in the form of a silicone gel.

Soft silicone adhesives are particularly suited for use as skin contact layers in wound dressings. They are soft, tactile and conformable, and exhibit good adhesion to dry skin but low adherence to an underlying wound. Thus, the dressing can be applied to a wound and subsequently removed without causing trauma to the wound. Silicone gels are adhesive but do not leave fibres or residue on a surface/substrate when removed.

Silicone gels suitable for use as skin contact materials in the present invention may be carried on a layer of melt-blown non-woven material, eg a sheet of melt-blown polyurethane (MBPU), as described in WO2007/113597. The reverse side of the MBPU may be coated with an adhesive, eg an acrylic adhesive, to affix the silicone gel/MBPU laminate to the overlying components of the dressing, eg the three-dimensional textile material and/or the backing layer.

The adhesive may be provided only at the border of the dressing, ie on the underside of the backing layer, around the three-dimensional textile material or overlapping the three-dimensional textile material to a small extent. Alternatively, however, the adhesive may extend across the entire extent of the underside of the wound dressing, covering the three-dimensional textile material. In such a case, the adhesive layer will generally be provided with openings, eg relatively large perforations, to allow the transfer of wound exudate through the adhesive layer and into the three-dimensional textile material. In such cases, the adhesive layer overlies and contacts the wound itself, rather than just the surrounding healthy skin, and so it is generally preferable that the adhesive that is used is one that is non-adherent and permits the wound dressing to be removed relatively easily and without causing trauma to the wound. Thus, the adhesive may be, for instance, a hydrocolloid adhesive, a polyurethane adhesive, a hydrogel or, most preferably, a soft silicone adhesive, particularly a silicone gel. The silicone gel may be carried on a sheet of MBPU, in which case the MBPU/silicone laminate may be formed with a regular array of relatively large perforations, eg perforations having a diameter of 2-10mm or more, eg 4-10mm. Perforations may also be provided in an adhesive layer which is present only at the border of the dressing. In such a case, the perforations will be relatively small, and are provided to increase breathability of the dressing.

The wound dressing may also comprise an absorbent body. The absorbent body will typically be positioned above the three-dimensional textile material, ie on the opposite side of the three-dimensional textile material to the wound, in order to absorb wound exudate that passes through the three-dimensional textile material. The absorbent body may have any suitable form and may be made of any suitable material known in the art. For instance, the absorbent body may be a foam pad, eg an open-cell polyurethane foam, or a pad of gauze or other woven or non-woven material. Other forms of absorbent body include an absorbent polymeric material. Suitable absorbent polymeric materials include polysaccharides or polysaccharide derivatives, and may be a material similar to those suitable for the production of the three-dimensional textile material. For example, the absorbent material may be alginate (ie a salt of alginic acid) and in particular sodium alginate or calcium alginate, or a blend of the two. Other suitable absorbent materials include cellulose, or a cellulose derivative such as hydroxyethyl cellulose or hydroxypropyl cellulose, and pectin or a pectin derivative such as amidated pectin.

Alternatively, the absorbent material may be of the type commonly referred to as a "superabsorber" or "superabsorbent material". Such materials are typically capable of absorbing many times their own mass of water (eg up to 200, 300, 400, 500 or more times their own mass of water). Preferred superabsorbent materials are polymeric superabsorbent materials and include polyacrylate (ie a salt of polyacrylic acid), polyacrylamide copolymers, ethylene maleic anhydride copolymer, carboxymethylcellulose, polyvinylalcohol copolymers, polyethylene oxide and starch-grafted copolymers of polyacrylonitrile.

Where the dressing comprises a composite structure with several components, eg the three-dimensional textile, an absorbent pad and the backing layer, those components may, where necessary or desired, by affixed together by conventional means, eg suitable adhesives.

Where the wound dressing includes a skin contact adhesive, the dressing will generally be supplied with a releasable liner on its underside. The releasable liner may cover the adhesive and wound contact portions of the wound dressing prior to use, and be removed from the dressing immediately before application of the dressing to the wound. This reduces the risk of contamination of the wound dressing and facilitates handling of the dressing.

Such releasable liners are commonly used on wound dressings known in the art, and suitable materials which can be employed in the present invention will be familiar to the skilled worker. For example, the releasable liner may be of a suitable plastics sheet or a siliconised paper or the like.

The releasable liner may be a single sheet which covers the underside of the wound dressing, or may be formed of two or more sheets. The releasable liner may further comprise a tab to enable the liner to be easily removed from the dressing before use. In particular, where the releasable liner is formed of two or more parts, the parts may either overlap or abut and extend outwards from the wound dressing, thus providing an easy method for removal of the releasable liner.

A composite wound dressing of this aspect of the disclosure may be of particular utility in negative pressure wound therapy.

Thus, according to another aspect of the disclosure, there is provided a wound dressing for use in negative pressure wound therapy, the wound dressing comprising an occlusive backing layer fitted with a drainage port, and a three-dimensional textile material comprising gelling fibres.

The three-dimensional textile material and wound dressing may have any of, or any combination of, the features described above in relation to other aspects of the disclosure. The backing layer is occlusive, to permit negative pressure to be applied to the wound. Having said that, the backing layer may have a degree or permeability to air and moisture vapour and so may be, for instance, a microporous polyurethane film as described above, but the backing layer should be sufficiently impermeable to allow negative pressure to be applied.

The drainage port is typically affixed to the backing layer of the wound dressing, the backing layer having an opening below the port to allow material to pass from the interior of the dressing through the drainage port.

Generally, the structure of the wound dressing will be such that, in use, the three-dimensional textile material is positioned in contact or communication with the wound. The backing layer of the dressing will extend over the three-dimensional textile material and will be sealingly affixed to healthy skin around the wound so that negative pressure may be applied between the dressing and the wound. One or more intermediate components, such as an absorbent body, may be interposed between the three-dimensional textile material and the backing layer of the dressing.

The drainage port is arranged such that a source of negative pressure can be connected to it. The source of negative pressure may be, for example, a pump. When a negative pressure is applied to the drainage port, a partial vacuum is formed under the wound dressing.

Also disclosed are composite dressings as described above, but which comprise a knitted structure according to the first aspect of the disclosure, rather than a three-dimensional textile material.

Additional agents known to assist or enhance wound healing may also be incorporated into any of the wound dressings according to the invention. For example, the dressing may also include antimicrobial agents, antiseptic agents, antifungal agents and/or anti-inflammatory agents. Such agents may be incorporated into the yarn prior to knitting, or may be added to the final dressing.

The wound dressings of the present invention may comprise honey. Honey has long been known to be effective in treating wounds, with records of such use dating from at least 2000 years ago. More recently, research has shown honey to have potent antimicrobial, antifungal and anti-inflammatory properties, and to be able to stimulate lymphocytic and phagocytic activity within the body. Further, honey has been demonstrated to assist in the debridement of necrotic tissue, and to stimulate the growth of new tissue. In terms of its antibacterial activity, honey has been reported to have an antibacterial effect on more than 60 species of bacteria, including aerobes, anaerobes, Gram-positive and Gram-negative bacteria. In particular, honey has been shown to be effective against antibiotic resistant strains of bacteria including MRSA.

Without wishing to be bound by theory, it is believed that the antibacterial activity of honey is partly due to the release of low levels of hydrogen peroxide, a well known antimicrobial agent. As the production of hydrogen peroxide is stimulated by dilution of the honey (eg by wound exudate), honey has the distinctive property of becoming more active on dilution, rather than less.

Many studies have shown that the maintenance of a moist wound environment aids in wound healing. However, a moist environment also promotes the growth of bacteria, and the prevention of infection is therefore a serious concern. The addition of honey to a wound dressing thus enables the wound to be kept moist whilst inhibiting bacterial growth and reducing the likelihood of infection.

In particular embodiments of the present invention, a wound dressing may comprise a knitted structure or three-dimensional textile material as described above, impregnated with honey or with honey on its surface. Alternatively, honey may be applied to the dressing or directly to the wound prior to application of the dressing.

Honey is produced worldwide from many different floral sources, and its antibacterial activity varies with the source of the honey and the processing it has undergone. For example, lotus honey in India is reputed to be good for eye diseases, whereas manuka honey, a monofloral honey produced from pollen from the manuka bush, is known for its antiseptic properties. The manuka plant is part of the genus *Leptospermum,* and honeys produced from plants of this genus, such as manuka or jellybush honey, are known for their particularly strong anti-bacterial properties. Preferably, the honey used in the present invention is produced from plants of the genus *Leptospermum.* More preferably, the honey is manuka honey or jellybush honey.

The wound dressings of the present invention may also comprise silver. Despite metallic silver being relatively unreactive, ionic silver has been shown to have antimicrobial activity and has been previously used in wound dressings. In use, positively charged silver ions bind to negatively charged sites on proteins and nucleic acids in bacteria. This causes a number of effects, including alteration of the protein structure, rupture of the cell wall and/or cell death. It is believed that silver ions have multiple attack sites, interacting with a number of different functional groups in bacteria, including thiol groups, carboxylates, phosphates, indoles and amines. This makes the development of bacterial resistance to silver unusual.

It is preferable that the incorporation of silver into the wound dressing results in the sustained release of low concentrations of silver ions over time. Such a slow release has been shown to stimulate healing and inhibit the growth of micro-organisms. Methods for incorporating silver into wound dressings are known in the art, as is the form in which silver may be used. Any suitable method or form known in the art may be used in the present invention. For example, the silver may be in the form of silver sulphadiazine.

Another antimicrobial that may be incorporated into the wound dressing and yarn according to the invention is polyhexamethylene biguanide (PHMB; also known as polyaminopropyl biguanide). PHMB is available as a 20% aqueous solution under the trade name COSMOCIL® CQ from Arch Personal Care Products, 70 Tyler Place, South Plainfield, NJ 07080, USA.

PHMB may be applied to a knitted structure or three-dimensional textile material according to the invention after that material has been produced. This may be achieved by spraying of an aqueous solution of PHMB onto the material, or by immersion of the material in such a solution.

In other embodiments, PHMB is applied to or incorporated into the yarn from which the material is produced. PHMB may be applied to the yarn by immersing the yarn in a solution of PHMB. PHMB may be incorporated into the yarn by including the PHMB in a solution used to produce the gelling fibre by solvent spinning, eg a solution of sodium alginate that is transformed into calcium alginate fibre by solvent-spinning through a solution of calcium ions, or a solution of pectin that is solvent-spun into a bath of a water-miscible organic solvent.

The concentration of antimicrobial agent such as silver or PHMB in the knitted structure or three-dimensional textile material according to the invention may vary between quite wide limits. However, the antimicrobial is typically present at a level of between 0.1 and 10% w/w in the material, more typically between 0.1 and 5% w/w, more commonly between 0.1 and 2% w/w or between 0.1 and 1% w/w.

The invention will now be described in greater detail, by way of example only, with reference to the accompanying drawings, in which
Figures 1(a) and 1(b) are lapping diagrams showing the structure of a fabric knitted using a yarn as disclosed herein;
Figure 2 shows a first, substantially square, embodiment of a wound dressing as disclosed herein;
Figure 3 shows a second, square, wound dressing similar to that of Figure 2, which has been impregnated with honey;
Figure 4 shows a third, tubular, embodiment of a wound dressing as disclosed herein;
Figure 5 shows an embodiment of a wound dressing according to the invention, in the form of an alginate foam reinforced with a knitted scrim comprising a yarn according to the disclosure;
Figure 6 is a perspective view, schematic and not to scale, of a spacer fabric comprising gelling fibres that constitutes a fifth embodiment of a wound dressing according to the disclosure;
Figure 7 is a perspective view from above of a sixth embodiment of a wound dressing according to the disclosure, incorporating a gelling spacer fabric of the form shown in Figure 6;
Figure 8 is an underside plan view of the wound dressing of Figure 7;
Figure 9 is across-sectional view, not to scale, on the line VIII-VIII in Figure 8;
Figure 10 is a view similar to Figure 9 of a seventh embodiment of a wound dressing according to the disclosure, incorporating a gelling spacer fabric and adapted for use in negative pressure wound therapy (NPWT);
Figure 11 is a view similar to Figure 10 of a eighth embodiment of a wound dressing suitable for NPWT, with a silicone gel skin contact layer that extends across the full extent of the underside of the dressing;
Figure 12 is an underside plan view of the wound dressing of Figure 11, with the release liners removed; and
Figure 13 is a view similar to Figures 11 and 12 of an ninth embodiment of a wound dressing according to the disclosure, again suitable for NPWT; and
Figure 14 is an underside plan view of the wound dressing of Figure 13, with the release liners removed.

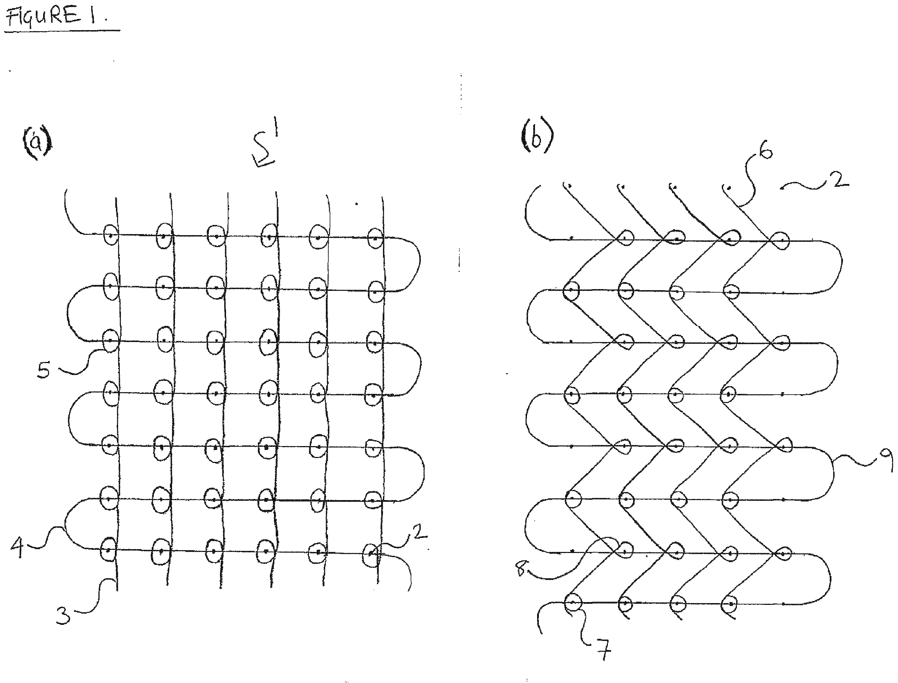

Referring first to Figure 1(a), there is shown a lapping diagram 1 of the pattern for a warp-knitted fabric. The lapping diagram is formed of a grid of dots. Each dot represents a needle head 2, and the lines 3 and 4 represent the path of the yarn in the knitted fabric. Each horizontal row of needle heads represents one course, ie a single stitch forming process. Each vertical column of needle heads is related to an individual yarn 3 (a warp yarn), and each of these individual yarns travels vertically up the column, forming a closed loop 5 about each needle head 2. The lapping diagram also includes a single weft yarn 4 which runs horizontally across each course, interlocking with the closed loops 5 of the warp yarn 3 and thereby forming the fabric structure. The resulting fabric has a crochet stitch pattern.

Figure 1(b) is a lapping diagram showing an alternative structure for a warp-knitted fabric, formed in a closed tricot stitch pattern with a two needle warp. Each vertical column of needle heads is related to an individual yarn 6 (a warp yarn). The yarn forms a closed loop 7 around the first needle head in the column. It then travels upwards, and forms a closed loop around a needle head in the row above, but in the adjacent column 8. The yarn then travels up, and forms a closed loop around a needle head in the third row, but in the first column. The yarn continues upward in this manner, forming a zig-zag pattern. The lapping diagram also includes a single weft yarn 9 which runs horizontally across each course, interlocking with the closed loops of the warp yarn and thereby forming the fabric structure. It is the system of interlocking loops that gives the knitted fabric its tensile strength, as well as a degree of elasticity.

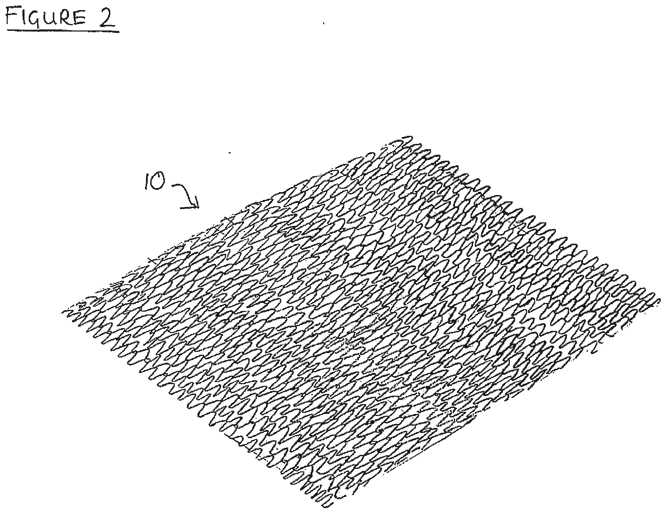

The lapping diagrams of Figure 1 can be used to produce a knitted fabric. As shown in Figure 2, such a knitted fabric may be produced as a square, and forms a 10cm x 10cm square wound dressing 10.

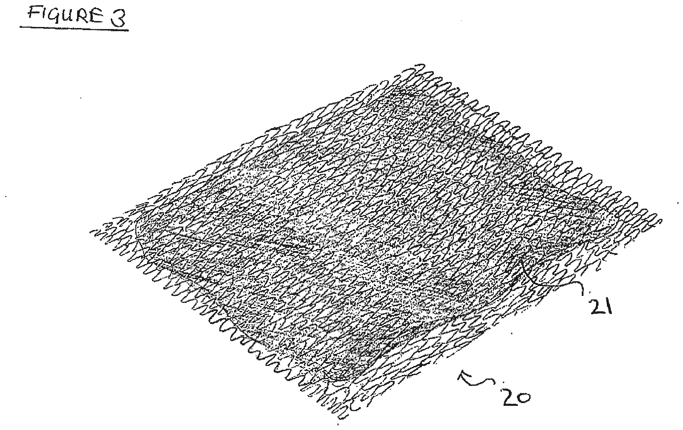

Figure 3 shows a further embodiment of the disclosure in which a square wound dressing 20 (similar to the dressing 10 of Figure 2) has been dosed with 25g of manuka honey 21. The honey 21 is deposited on the surface of the wound dressing 20, and is substantially fully absorbed into the knitted substrate. The honey extends irregularly over the extent of the wound dressing 20, covering a substantial portion of the surface area.

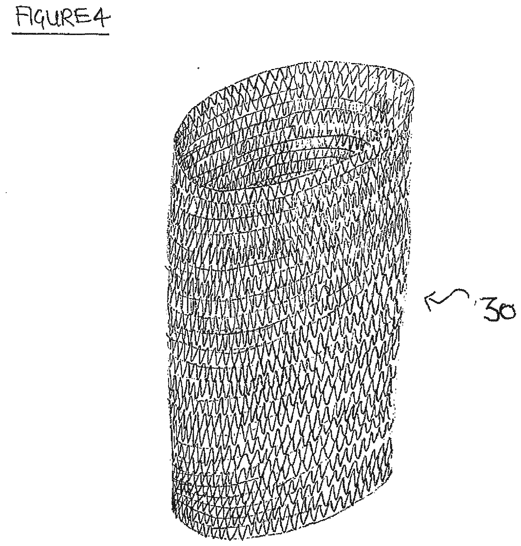

Figure 4 shows a tubular dressing 30, of the type worn on an arm or a leg, produced using fabric knitted to the pattern of Figure 1(a) or 1(b). The tubular dressing 30 has been produced by knitting in the round, so that there is no seam running lengthways down the dressing.

Alternatively, the tubular dressing 30 may be produced from a flat sheet of knitted material which is sewn together along opposite edges to form a tube. The tubular dressing may be produced as an individual unit, or as an extended tube which may be cut to the desired size.

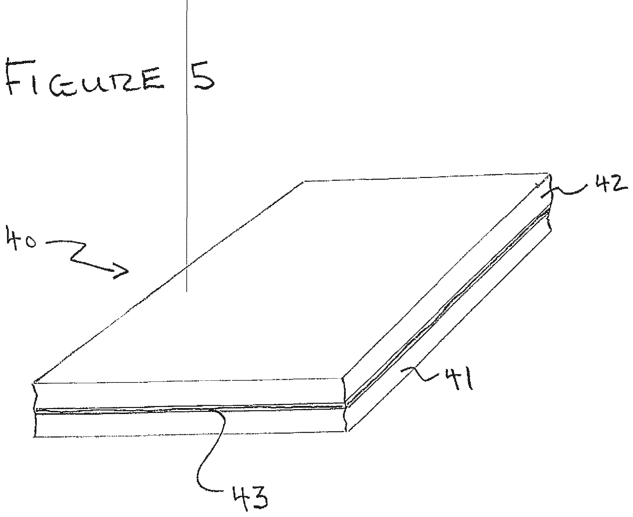

Figure 5 shows an embodiment of a wound dressing according to the invention, in this case an alginate foam dressing 40. The dressing 40 comprises first and second layers 41,42 of alginate foam that are reinforced by a sheet of knitted fabric 43 (similar to the sheet 10 of Figure 2) that is sandwiched between the layers 41,42 of foam.

The dressing 40 is manufactured by casting a first quantity of alginate solution (typically containing sodium alginate, a source of calcium ions, and a sequestering agent to prevent premature precipitation of calcium alginate) into a rectangular mould. The solution is allowed to partially cure and the sheet of knitted fabric 43 is laid on top of the partially cured solution. A second quantity of alginate solution is then cast on top of the knitted fabric 43, and curing of both quantities of solution is allowed to continue until hydrogels are formed on both sides of the knitted fabric 43. The product is then subjected to lyophilisation to remove water from the hydrogels and to create the foam layers 41,42.

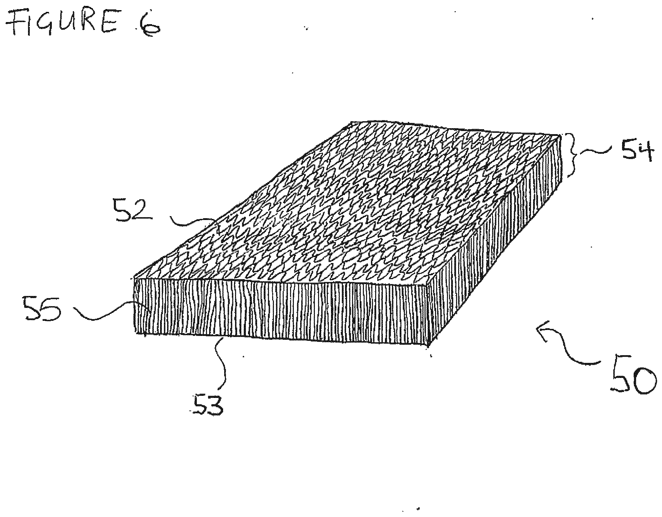

Referring now to Figure 6, a spacer fabric 50 comprises an upper face layer 52, a lower face layer 53, and a connecting layer 54 which comprises connecting threads 55 that extend between the upper face layer 52 and lower face layer 53. The spacer fabric is knitted by a conventional process suitable for the production of such a knitted structure, using a yarn that comprises a blend of gelling fibres and non-gelling cellulosic fibres. The gelling fibres are present in a major proportion (approximately 90%), the minor proportion of non-gelling fibres serving to maintain the integrity of the fabric even after gelling of the gelling fibres.

If necessary or desired, for instance to minimise risk of fibre fragments being lost from the fabric 50 in use, at the edges of the spacer fabric 50, the upper face layer 52, the lower face layer 53 and the connecting layer 54 may be fused together to create a seal.

The spacer fabric 50 of Figure 6 may be used as a simple form of wound dressing, in the manner of a conventional gelling dressing, such as those that have the form of a non-woven pad of gelling alginate or carboxymethylcellulose fibres. Such dressings are simply applied to the wound surface and absorb wound exudate. As the exudate is absorbed, the fibres are transformed into a gel that maintains a moist environment at the wound surface and is non-adherent, which enables the dressing to be removed without trauma to the wound.

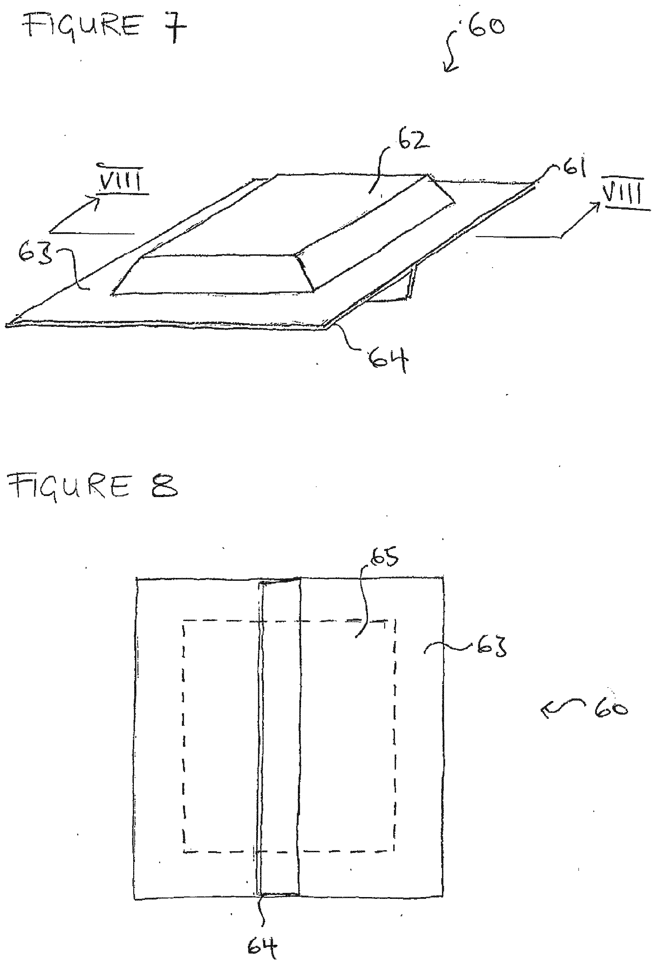

Alternatively, the spacer fabric 50 of Figure 1 may form a component of a composite wound dressing. Figures 7 to 14 illustrate embodiments of such composite wound dressings.

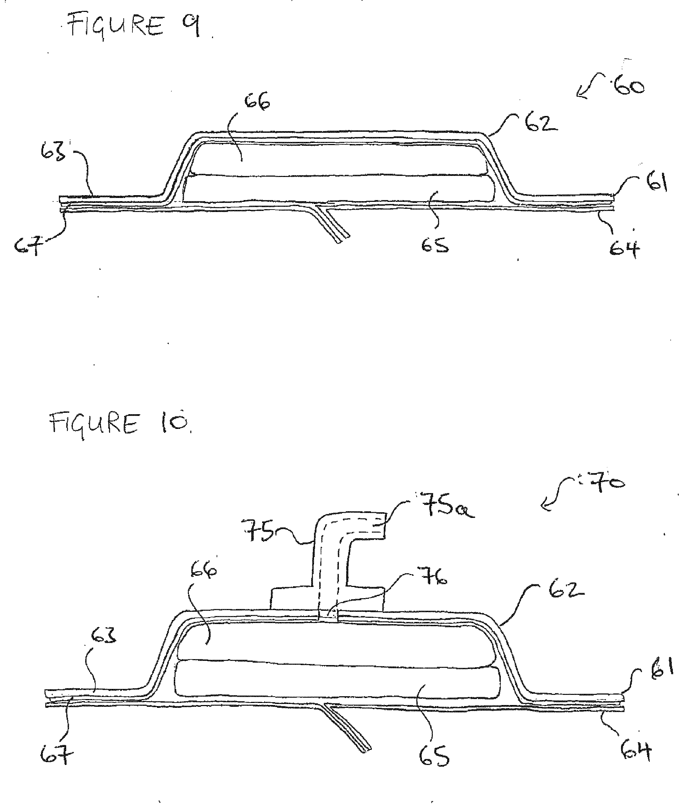

The wound dressing of Figures 7 to 9 is generally designated 60 and is generally square in form and comprises a backinglayer 61 of microporous polyurethane film, having a central portion 62 and a border 63. The central portion 62 overlies a composite body comprising a square piece of three-dimensional textile material 65 similar to that shown in Figure 6 and an absorbent foam pad 66. The textile material 65 and foam pad 66 are affixed to each other by any suitable means, eg by adhesive. The three-dimensional textile 65 is shown as hidden detail in Figure 8 and the composite is visible in Figure 9.

The underside of the backing layer 61 carries a coating of adhesive 67, eg an acrylic adhesive, by means of which the backing layer is affixed at its central portion 62, to the absorbent pad 66. The adhesive coating 67 on the border 63 serves to attach the dressing 60 to the skin surrounding a wound, so that in use the three-dimensional textile 65 overlies the wound. The wound dressing 60 is supplied with a two-part release liner 64, which is removed from the dressing immediately prior to use, in order to expose the three-dimensional textile material 65 and the adhesive 67 on the underside of the border 63. The underside of the wound dressing 60 is depicted in Figure 8, in which the three-dimensional textile material 1652 is shown in broken lines as hidden detail.

Figure 9 is a cross-sectional view of the wound dressing 60 described above. The wound dressing includes a composite body that comprises the three-dimensional textile material 65 and absorbent foam pad 66. The three-dimensional textile material 65 and the absorbent pad 66 are overlaid by the backing layer 61 of polyurethane film, the absorbent pad 66 being secured to the underside of the backing layer 61 by the coating of adhesive 67 . The backing layer 61 extends beyond the edges of the three-dimensional textile material 65 and the absorbent pad 66 on all sides to form the border 63 that is also coated with the adhesive 67. The releasable liner 64, which comprises two sheets of suitable plastics film or other conventional material that overlap to form tabs to facilitate removal of the liner 64, covers the adhesive 67 and the exposed face of the three-dimensional textile material 65. The releasable liner 64 is removed immediately prior to use.

Figure 10 shows a further embodiment of a wound dressing, generally designated 70, according to the invention that is suitable for use in negative pressure wound therapy. This dressing 70 is broadly similar in construction to that of Figures 7 to 9, and corresponding components of the dressing are denoted by the same reference numerals. Thus, the dressing 70 is generally square in form and comprises a backinglayer 61 of microporous polyurethane film, having a central portion 62 and a border 63. The central portion 62 overlies a composite body comprising a square piece of three-dimensional textile material 65 similar to that shown in Figure 6 and an absorbent foam pad 66. The textile material 65 and foam pad 66 are affixed to each other by any suitable means, eg by adhesive. The three-dimensional textile 65 is shown as hidden detail in Figure 8 and the composite is visible in Figure 9.

The underside of the backing layer 61 carries a coating of adhesive 67, eg an acrylic adhesive, by means of which the backing layer is affixed at its central portion 62, to the absorbent pad 66. The adhesive coating 67 on the border 63 serves to attach the dressing 60 to the skin surrounding a wound, so that in use the three-dimensional textile 65 overlies the wound. The wound dressing 60 is supplied with a two-part release liner 64, which is removed from the dressing immediately prior to use, in order to expose the three-dimensional textile material 65 and the adhesive 67 on the underside of the border 63.

The wound dressing 70 includes a composite body that comprises the three-dimensional textile material 65 and absorbent foam pad 66. The three-dimensional textile material 65 and the absorbent pad 66 are overlaid by the backing layer 61 of polyurethane film, the absorbent pad 66 being secured to the underside of the backing layer 61 by the coating of adhesive 67 . The backing layer 61 extends beyond the edges of the three-dimensional textile material 65 and the absorbent pad 66 on all sides to form the border 63 that is also coated with the adhesive 67. The releasable liner 64, which comprises two sheets of suitable plastics film or other conventional material that overlap to form tabs to facilitate removal of the liner 64, covers the adhesive 67 and the exposed face of the three-dimensional textile material 65. The releasable liner 64 is removed immediately prior to use.

The dressing 70 differs from the dressing 60 in that the backing layer 61 is provided with a central opening 76 and a drainage port 75 is affixed to the backing layer 61 directly above the opening 76. The drainage port 75 is formed by injection moulding in plastics material and includes an upstand with a central bore 75a that is aligned with the opening 76.

The dressing 70 is applied to a wound in the same manner as the dressing 60. The dressing 70 can be used in NPWT by attaching (by means of a flexible tube) the drainage port 75 to a source of reduced pressure, such as a pump. The pressure within the interior of the dressing 70 is thereby reduced to less than the ambient air pressure. Excess wound exudate is drawn through the three-dimensional textile material 65, the absorbent pad 66 and the drainage port 75, and is collected in a suitable receptacle in a conventional manner.

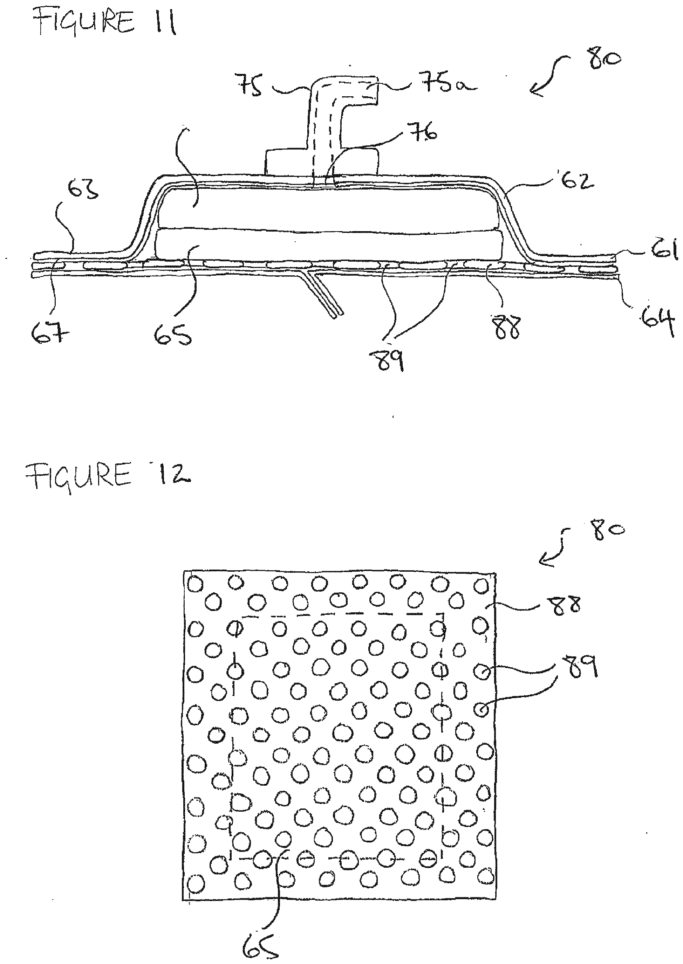

A further embodiment of a wound dressing, generally designated 80 and again suitable for use in NPWT, is shown in Figures 11 and 12. This dressing 80 is broadly similar to the dressing 70 of Figure 10, and again corresponding components of the dressing are denoted by the same reference numerals.

In the dressing 80, however, a silicone gel skin contact layer 88 extends across the whole extent of the underside of the dressing 80. The skin contact layer 88 comprises a sheet of melt-blown polyurethane that carries a coating of silicone gel. The reverse side of the melt-blown polyurethane is coated with acrylic adhesive by which it is affixed to the border 63 of the backing layer 61 and to the underside of the three-dimensional textile material 65. The skin contact layer is formed with a regular array of perforations 89 with approximate diameter 5mm that permit wound exudate to pass from the wound into the three-dimensional textile material 65.

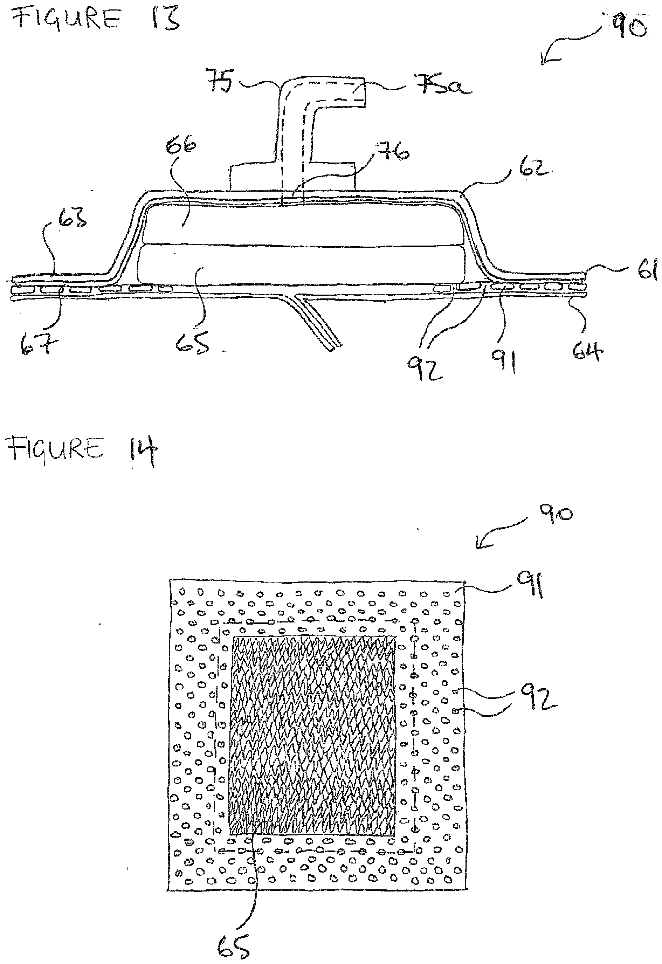

Finally, an embodiment of a wound dressing, generally designated 90 and again suitable for use in NPWT, is shown in Figures 13 and 14. This dressing 90 is broadly similar to the dressing 80 of Figure 11, and corresponding components of the dressing are denoted by the same reference numerals.

In the dressing 90, however, the silicone gel layer 91 is present around the border 63 of the dressing 90 and partially overlaps the three-dimensional textile material 65. In other words, the silicone gel layer 91 is formed with a central opening that is slightly smaller in size than the three-dimensional textile material 65, so that the majority of the textile material 65 is exposed and, in use, directly contacts the wound. The composition of the silicone gel layer 91 is the same as that of the skin contact layer 88 as described in relation to Figure 11, with the exception that the perforations 92 are of smaller diameter. In use, the silicone gel layer 91 contacts the skin around the edge of the wound, and the three-dimensional textile material 65 contacts the wound directly.

## Claims

1. A gelling foam dressing, comprising a sheet of gelling foam laminated with a scrim comprising gelling fibres and non-gelling fibres.

2. The gelling foam dressing of Claim 1, wherein the scrim is a knitted structure which is knitted from yarn comprising a blend of gelling fibres and non-gelling fibres, wherein the yarn comprises at least 50% w/w gelling fibres.

3. The gelling foam dressing of claim 2, wherein the gelling fibres are fibres selected from the group consisting of pectin fibres, alginate fibres, fibres made from alginate and another polysaccharide, chitosan fibres, hyaluronic acid fibres, fibres of other polysaccharides or derived from gums and chemically-modified cellulosic fibres, or combinations thereof.

4. The gelling foam dressing of Claim 3, wherein the gelling fibres are alginate fibres and/or pectin fibres.

5. The gelling foam dressing of Claim 4, wherein the gelling fibres are calcium alginate.

6. The gelling foam dressing of any one of the preceding claims, wherein the non-gelling fibres are cellulosic fibres.

7. The gelling foam dressing of Claim 6, wherein the cellulosic fibres are lyocell or viscose.

8. The gelling foam dressing of any one of the preceding claims, which comprises one of calcium alginate and viscose; calcium alginate and lyocell; and pectin and lyocell.

9. The gelling foam dressing of Claim 1, wherein the scrim is woven.

10. The gelling foam dressing of any of Claims 1-9, wherein the gelling material used in the gelling foam dressing is alginate.

11. The gelling foam dressing of any of Claims 1-10, further comprising one or more additional agents selected from antimicrobial agents, antiseptic agents, antifungal agents and/or anti-inflammatory agents.

12. The gelling foam dressing of any of Claims 1-11, comprising a single sheet of foam, to one face of which the scrim is laminated.

13. A method of manufacturing a gelling foam dressing according to Claim 12, comprising:
a) casting a solution of gelling material into a mould,
b) allowing the solution to partially cure,
c) laying the scrim on top of the partially-cured solution,
d) allowing the solution to cure further to form a hydrogel, and
e) removing water from the hydrogel to form a foam sheet bonded to the scrim.

14. The gelling foam dressing of any of Claims 1-11, wherein the scrim is sandwiched between layers of foam.

15. A method of manufacturing a gelling foam dressing according to Claim 14, comprising:
a) casting a first quantity of solution of gelling material into a mould,
b) allowing the solution to partially cure,
c) laying the scrim on top of the partially-cured solution,
d) casting a further quantity of a solution of gelling material onto the scrim,
e) allowing the solution to cure further to form a hydrogel, and
f) removing water from the hydrogel to form foam sheets bonded to both sides of the scrim.

## Patentansprüche

1. Gelierender Schaumstoffverband, der eine Bahn aus gelierendem Schaumstoff umfasst, die mit einem Gitterstoff laminiert ist, der gelierende Fasern und nichtgelierende Fasern umfasst.

2. Gelierender Schaumstoffverband nach Anspruch 1, wobei der Gitterstoff eine gestrickte Struktur ist, die aus einem Garn gestrickt ist, die ein Gemisch von gelierenden Fasern und nichtgelierenden Fasern umfasst, wobei das Garn mindestens 50 Gew.-% gelierende Fasern umfasst.

3. Gelierender Schaumstoffverband nach Anspruch 2, wobei es sich bei den gelierenden Fasern um Fasern handelt, die aus der Gruppe bestehend aus Pektinfasern, Alginatfasern, aus Alginat und einem anderen Polysaccharid hergestellten Fasern, Chitosanfasern, Hyaluronsäurefasern, Fasern von anderen Polysacchariden oder von Gummen abgeleiteten Fasern und chemisch modifizierten Cellulosefasern oder Kombinationen davon ausgewählt sind.

4. Gelierender Schaumstoffverband nach Anspruch 3, wobei die gelierenden Fasern Alginatfasern und/oder Pektinfasern sind.

5. Gelierender Schaumstoffverband nach Anspruch 4, wobei die gelierenden Fasern Calciumalginat sind.

6. Gelierender Schaumstoffverband nach einem der vorhergehenden Ansprüche, wobei die nichtgelierenden Fasern Cellulosefasern sind.

7. Gelierender Schaumstoffverband nach Anspruch 6, wobei die Cellulosefasern Lyocell oder Viskose sind.

8. Gelierender Schaumstoffverband nach einem der vorhergehenden Ansprüche, der eines von Calciumalginat und Viskose; Calciumalginat und Lyocell; und Pektin und Lyocell umfasst.

9. Gelierender Schaumstoffverband nach Anspruch 1, wobei der Gitterstoff gewebt ist.

10. Gelierender Schaumstoffverband nach einem der Ansprüche 1-9, wobei das in dem gelierenden Schaumstoffverband verwendete gelierende Material Alginat ist.

11. Gelierender Schaumstoffverband nach einem der Ansprüche 1-10, der weiterhin ein oder mehrere zusätzliche Mittel umfasst, die aus antimikrobiellen Mitteln, antiseptischen Mitteln, antimykotischen Mitteln und/oder entzündungshemmenden Mitteln ausgewählt sind.

12. Gelierender Schaumstoffverband nach einem der Ansprüche 1-11, der eine einzige Schaumstoffbahn umfasst, wobei der Gitterstoff auf einer Fläche dieser laminiert ist.

13. Verfahren zur Herstellung eines gelierenden Schaumstoffverbands nach Anspruch 12, umfassend:
a) Gießen einer Lösung eines gelierenden Materials in eine Gussform,
b) partielles Aushärtenlassen der Lösung,
c) Legen des Gitterstoffs auf die partiell ausgehärtete Lösung,
d) weiteres Aushärtenlassen der Lösung, um ein Hydrogel zu bilden, und
e) Abziehen von Wasser aus dem Hydrogel, um eine Schaumstoffbahn zu bilden, die an den Gitterstoff gebondet ist.

14. Gelierender Schaumstoffverband nach einem der Ansprüche 1-11, wobei der Gitterstoff zwischen Schaumstoffschichten eingeschoben ist.

15. Verfahren zur Herstellung eines gelierenden Schaumstoffverbands nach Anspruch 14, umfassend:
a) Gießen einer ersten Menge einer Lösung eines gelierenden Materials in eine Gussform,
b) partielles Aushärtenlassen der Lösung,
c) Legen des Gitterstoffs auf die partiell ausgehärtete Lösung,
d) Gießen einer weiteren Menge einer Lösung eines gelierenden Materials auf den Gitterstoff,
e) weiteres Aushärtenlassen der Lösung, um ein Hydrogel zu bilden, und
f) Abziehen von Wasser aus dem Hydrogel, um Schaumstoffbahnen zu bilden, die an beide Seiten des Gitterstoffs gebondet sind.

## Revendications

1. Pansement en mousse gélifiante, comprenant une feuille de mousse gélifiante stratifiée avec un canevas comprenant des fibres gélifiantes et des fibres non gélifiantes.

2. Pansement en mousse gélifiante selon la revendication 1, dans lequel le canevas est une structure tricotée qui est confectionnée à partir de fils comprenant un mélange homogène de fibres gélifiantes et de fibres non gélifiantes, dans lequel le fil comprend au moins 50 % p/p de fibres gélifiantes.

3. Pansement en mousse gélifiante selon la revendication 2, dans lequel les fibres gélifiantes sont des fibres choisies dans le groupe constitué des fibres de pectine, des fibres d'alginate, des fibres fabriquées à partir d'alginate et d'un autre polysaccharide, des fibres de chitosane, des fibres d'acide hyaluronique, des fibres d'autres polysaccharides ou dérivées de gommes et de fibres cellulosiques chimiquement modifiées, ou des combinaisons de celles-ci.

4. Pansement en mousse gélifiante selon la revendication 3, dans lequel les fibres gélifiantes sont des fibres d'alginate et/ou des fibres de pectine.

5. Pansement en mousse gélifiante selon la revendication 4, dans lequel les fibres gélifiantes sont l'alginate de calcium.

6. Pansement en mousse gélifiante selon l'une quelconque des revendications précédentes, dans lequel les fibres non gélifiantes sont des fibres cellulosiques.

7. Pansement en mousse gélifiante selon la revendication 6, dans lequel les fibres cellulosiques sont le lyocell ou la viscose.

8. Pansement en mousse gélifiante selon l'une quelconque des revendications précédentes, qui comprend l'un parmi l'alginate de calcium et la viscose ; l'alginate de calcium et le lyocell ; et la pectine et le lyocell.

9. Pansement en mousse gélifiante selon la revendication 1, dans lequel le canevas est tissé.

10. Pansement en mousse gélifiante selon l'une quelconque des revendications 1 à 9, dans lequel le matériau gélifiant utilisé dans le pansement en mousse gélifiante est l'alginate.

11. Pansement en mousse gélifiante selon l'une quelconque des revendications 1 à 10, comprenant en outre au moins un agent supplémentaire choisi parmi les agents antimicrobiens, les agents antiseptiques, les agents antifongiques et/ou les agents anti-inflammatoires.

12. Pansement en mousse gélifiante selon l'une quelconque des revendications 1 à 11, comprenant une feuille unique de mousse, sur une face de laquelle le canevas est stratifié.

13. Procédé de fabrication d'un pansement en mousse gélifiante selon la revendication 12, comprenant :
a) la coulée d'une solution de matériau gélifiant dans un moule,
b) le fait de permettre à la solution de durcir partiellement,
c) la pose du canevas sur la partie supérieure de la solution partiellement durcie,
d) le fait de permettre à la solution de durcir encore afin de former un hydrogel, et
e) l'élimination d'eau de l'hydrogel afin de former une feuille de mousse liée au canevas.

14. Pansement en mousse gélifiante selon l'une quelconque des revendications 1 à 11, dans lequel le canevas est pris en sandwich entre des couches de mousse.

15. Procédé de fabrication d'un pansement en mousse gélifiante selon la revendication 14, comprenant :
a) la coulée d'une première quantité de solution de matériau gélifiant dans un moule,
b) le fait de permettre à la solution de durcir partiellement,
c) la pose du canevas sur la partie supérieure de la solution partiellement durcie,
d) la coulée d'une quantité supplémentaire d'une solution de matériau gélifiant sur le canevas,
e) le fait de permettre à la solution de durcir encore afin de former un hydrogel, et
f) l'élimination d'eau de l'hydrogel afin de former des feuilles de mousse liées aux deux côtés du canevas.
